# EUROPÄISCHE PATENTSCHRIFT

(11) **EP 2 951 278 B1**
(45) Veröffentlichungstag und Bekanntmachung des Hinweises auf die Patenterteilung: **08.03.2017**
(21) Anmeldenummer: 14707225.0
(22) Anmeldetag: 27.01.2014
(51) Int. Cl.: C12M 1/107, C12M 1/12, C12M 1/00, C12M 3/00

(54) **BIOREAKTORAUFHÄNGUNG**
BIOREACTOR SUSPENSION
SUSPENSION DE BIORÉACTEUR

(30) Priorität: 29.01.2013 DE 102013001444
(43) Veröffentlichungstag der Anmeldung: 09.12.2015
(73) Patentinhaber: Pateffect Schutzrechtsmanagement GBR, 73430 Aalen (DE)
(72) Erfinder: VÄTH, Hans, 64409 Messel (DE)
(86) Internationale Anmeldenummer: PCT/IB2014/058584
(87) Internationale Veröffentlichungsnummer: WO 2014/118690

(56) Entgegenhaltungen:
- EP-A1- 0 402 496
- WO-A1-2010/109108
- AU-A4- 2006 100 045
- CN-A- 101 597 567
- DE-A1- 19 814 253
- DE-A1-102004 007 564
- US-A1- 2010 159 579

## Beschreibung

Die Erfindung betrifft eine Stützvorrichtung mit einer Vielzahl parallel und etwa horizontal verlaufender, sehr langgestreckter Bioreaktor-Elemente mit einer zumindest teilweise transparenten Wandung aus Kunststoff, durch welche eine mit Mikroorganismen beimpfte Nährmittellösung zur Herstellung von Biomasse mittels Photosynthese geführt wird, mit mehreren entlang der Bioreaktor-Elemente angeordneten, horizontal voneinander beabstandeten, feststehenden Stützmasten.

Eine derartige Vorrichtung wird beispielsweise in der WO 2010/109108 A1 offenbart, welche allerdings zur Abstützung sehr vieler, langer Röhren ungeeignet ist.

Die Herstellung von Biomasse mittels Photosynthese ist seit längerer Zeit bekannt und wird u.a. in der DE 198 14 253 beschrieben.
Dabei kommen zunehmend geschlossene Systeme in Form von Röhrenreaktoren zum Einsatz, die direkt oder indirekt über Lichtleitsysteme mit Sonnenlicht oder künstlichem Licht bestrahlt werden.
In geschlossenen Systemen lassen sich die Prozessbedingungen wie Nährstoffgehalt, Temperatur, Lichtintensität und CO₂-Versorgung wesentlich genauer steuern als in offenen Anordnungen.
Im Interesse einer kostengünstigen Konstruktion werden die transparenten Rohre oder flexiblen Schläuche hierbei meist horizontal verlegt.
Beim Übergang zu Großanlagen erweist sich die sichere Abstützung und Führung der sehr lang gestreckten Bioreaktor-Elemente als relativ aufwendig.

Die Aufgabe der Erfindung ist es daher den Herstellungs- und Montageaufwand für derartige Stützvorrichtungen zu vermindern.

Erfindungsgemäß wurde die Aufgabe dadurch gelöst, dass über den jeweils zu tragenden Bioreaktor-Elementen wenigstens ein Tragelement zwischen zumindest zwei Stützmasten verläuft, das Tragelement sich auf den entsprechenden Stützmasten abstützt und die zu tragenden Bioreaktor-Elemente über wenigstens ein Halteelement an mindestens einem, zwischen den beiden Stützmasten liegenden Abstützpunkt mit zumindest einem Tragelement verbunden sind.
Durch die Realisierung von Abstützpunkten zwischen den Stützmasten kann der Abstand zwischen den Stützmasten erheblich vergrößert werden, was sich entsprechend auf deren erforderliche Anzahl und somit auch auf die Kosten auswirkt.

Daher entfaltet die Erfindung ihre besonderen Kostenvorteile, wenn die im Wesentlichen waagerecht verlaufenden Bioreaktor-Elemente eine Länge von mehr als 25, vorzugsweise mehr als 50 und insbesondere mehr als 100 m haben. Hierzu genügt es völlig, wenn der Abstand zwischen zwei benachbarten Stützmasten mindestens 3, vorzugsweise wenigstens 6 m und/oder der Abstand zwischen zwei benachbarten Abstützpunkten zumindest 1, vorzugsweise wenigstens 2 m beträgt.

Um die Herstellung sowie die Montage der Bioreaktor-Elemente zu vereinfachen, sollten diese jeweils einstückig ausgebildet sein. Hierbei ist es vorteilhaft, wenn die Bioreaktor-Elemente in der Nähe ihres Einsatzortes mit Hilfe einer mobilen Herstellungseinheit erzeugt werden.

Die Abstützung der Bioreaktor-Elemente erfolgt in den Abstützpunkten indirekt über die Halteelemente und die Tragelemente auf den benachbarten Stützmasten. Dieser Vorteil wird insbesondere dann klar, wenn in ihrer einfachsten Form das Tragelement von einem zwischen den Stützmasten verlaufenden, flexiblen Stützseil, Stützband o.ä. und die Haltelemente von einfachen Haken gebildet werden.

Neben den Abstützpunkten sollten sich die Bioreaktor-Elemente ergänzend direkt auf den Stützmasten, vorzugsweise auf, an den Stützmasten befestigten Stützarmen abstützen.
Wegen der hier angestrebten, großen Längenabmessung der Bioreaktor-Elemente werden diese meist am Beginn der Montage am Boden in Position gebracht. Um die endgültige Positionierung zu erleichtern, ist es von Vorteil, wenn die Stützmasten Hubvorrichtungen besitzen, welche geeignet sind, die Bioreaktor-Elemente in die vorgesehene Höhe, insbesondere in den Bereich des entsprechenden Stützarmes zu bringen.
Dabei kann die Montage noch dadurch erleichtert werden, dass die Stützarme in den Hubbereich der Hubvorrichtungen verlängerbar sind. Sind die Bioreaktor-Elemente auf den Stützarmen in ihrer Endposition, so können die Stützarme wieder verkürzt werden.

Des Weiteren ist es vorteilhaft, wenn die Ausdehnung der Halteelemente veränderbar ist. Dies erlaubt nicht nur ein Anheben der Bioreaktor-Elemente über die Halteelemente selbst, sondern auch eine Nachjustierung der Position der Bioreaktor-Elemente durch ein Verlängern oder Verkürzen des entsprechenden Halteelementes.

Je nach Anzahl und Gewicht der Bioreaktor-Elemente kann es vorteilhaft sein, wenn über jedem Bioreaktor-Element ein Tragelement verläuft. Um dabei den Aufwand zu begrenzen, ist es ebenso von Vorteil, wenn an zumindest einem, vorzugsweise allen Tragelementen jeweils mehrere Bioreaktor-Elemente befestigt sind.

Zur Erleichterung der Befestigung der Bioreaktor-Elemente an den Halteelementen sollten die Bioreaktor-Elemente im Bereich der Abstützpunkte Nuten, Ösen o.ä. zur Verbindung mit dem entsprechenden Halteelement aufweisen.

Alternativ ist es aber auch möglich, dass mehrere benachbarte Bioreaktor-Elemente im Bereich wenigstens eines Abstützpunktes über jeweils ein Koppelelement miteinander verbunden sind und das Koppelelement über ein oder mehrere Halteelemente an einem oder mehreren Tragelementen befestigt ist.

Im Interesse einer umfassenden Abstützung sollten sich die Tragelemente zumindest annähernd über die gesamte Länge der Bioreaktor-Elemente erstrecken.

Dabei kann eine besonders kompakte und einfache Konstruktion dadurch erreicht werden, dass die Bioreaktor-Elemente in mehreren übereinander liegenden, vorzugsweise horizontal verlaufenden Reihen angeordnet sind.

In diesem Fall sollte sich zumindest eine, vorzugsweise jede, auf einer Seite der Stützmasten verlaufende Reihe von Bioreaktor-Elementen auf jeweils einem Stützarm der Stützmasten abstützen.

Nachfolgend soll die Erfindung an mehreren Ausführungsbeispielen näher erläutert werden.
In der beigefügten Zeichnung zeigt:
Figur 1: eine Anlage zur Herstellung sehr langer Bioreaktor-Elemente 1;
Figur 2: einen Querschnitt durch eine Stützvorrichtung;
Figur 3: eine Längsdarstellung der Stützvorrichtung gemäß Figur 2;
Figur 4: einen Querschnitt durch eine andere Stützvorrichtung;
Figur 5: eine Längsdarstellung der Stützvorrichtung gemäß Figur 4 und
Figur 6: eine Detaildarstellung des Koppelelementes 7 gemäß Figur 4 und 5.

Die waagerecht verlaufenden Bioreaktor-Elemente 1 dienen der Produktion von phototropen Organismen und Zellkulturen unter Lichteinwirkung, weshalb diese aus einem ganz oder teilweise transparenten Kunststoff gefertigt werden.

Zur Herstellung sehr langer, einstückiger Bioreaktor-Elemente 1 eignet sich insbesondere das Extrusionsverfahren, bei dem eine feste bis dickflüssige und härtbare Masse unter Druck kontinuierlich aus einer formgebenden Düse des Extruders 10 gepresst. Dabei können komplexe Strukturen gebildet werden und weiche wie auch spröde Materialien verarbeitet werden.
Wegen der umfassenden Einsatzmöglichkeiten wird die zur Herstellung der Bioreaktor-Elemente 1 verwendete Extrusionsmasse hier von thermoplastischem Kunststoff, insbesondere PMMA, PC, PE, PET oder PVC gebildet.
Nach dem Austritt aus der Düse erstarrt der Kunststoff in einer folgenden und meist wassergekühlten Kalibrierung des Extruders 10.

Zur Bildung von Rohr- oder Profil-Elementen besitzt die Kalibrierung oft eine Kalibrierhülse, die dem Formerhalt des Bioreaktor-Elementes 1 dient. Dabei wird das Bioreaktor-Element 1 über Vakuum an die Innenwand der Kalibrierhülse gesaugt. Falls das Bioreaktor-Element 1 ein- oder mehrere Kanäle 8,9 besitzt, so können diese auch am, den Extruder 10 verlassende Ende luftdicht verschlossen werden. So ist es möglich, im Inneren der Kanäle 8,9 während der Herstellung des Bioreaktor-Elements 1 vom Extrusionswerkzeug des Extruders 10 her einen Luftüberdruck zu erzeugen und so das Bioreaktor-Element 1 von innen zu stützen. Dies kann insbesondere bei der Herstellung von Bioreaktor-Elementen 1 mit geringer Eigenstabilität erforderlich sein.

Nach der Kalibrierung schließt sich in der Regel noch eine Abzugsvorrichtung des Extruders 10 an, die das Bioreaktor-Element 1 beispielsweise über sich in Transportrichtung bewegende und gegen das Bioreaktor-Element 1 gedrückte Laufflächen aus dem Extruder 10 befördert.

Um die Transportprobleme bei sehr langen Bioreaktor-Elementen 1 zu vermeiden und die Herstellung sehr langer Bioreaktor-Elemente 1 insbesondere auch mit mehreren Kanälen 8,9 zu erleichtern, erfolgt deren Herstellung in unmittelbarer Nähe ihres Einsatzortes.
Hierbei wirkt außerhalb des Extruders 10 auf das, den Extruder 10 verlassende Ende eine Zugkraft ein, welche den Beginn des Bioreaktor-Elementes 1 vom Extruder 10 wegzieht.
Auf diese Weise können problemlos und kostengünstig Bioreaktor-Elemente 1 mit einer Länge von mehr als 25 m oder gar mehr als 50 m hergestellt werden. Durch die einstückige Fertigung großer Längen entfallen die Abdichtungsprobleme, die sich beim Zusammensetzen aus einzelnen Segmenten ergeben würden.
Dabei kann die Strecke nach dem Extruder 10, d.h. auch nach der Abzugsvorrichtung zur Abkühlung des Bioreaktor-Elementes 1 genutzt werden.

Damit der Extruder 10 trotz der Herstellung vor Ort umfassend einsetzbar ist, sollte dieser mobil ausgeführt sein. Hierzu bietet sich die Montage des Extruders 10 auf einem LKW an, von dessen Ladefläche dann der Beginn des Bioreaktor-Elementes 1 weggezogen wird. Im Ergebnis soll durch das Ziehen des Bioreaktor-Elementes 1 dieses möglichst nahe zum Einbauort gebracht werden.

Um die Zugkraft auf das den Extruder 10 verlassende Ende übertragen zu können, wird an diesem beispielhaft ein Koppelstück befestigt, welches über ein Seil mit einer als Zugvorrichtung 12 wirkenden Seilwinde verbunden ist.
Zur Vermeidung einer Überdehnung oder gar Beschädigung des Elements 1 infolge zu großer Kräfte, wird die Zugkraft von einer Zugvorrichtung 12 aufgebracht, deren Zugkraft und/oder Position veränderbar ist.

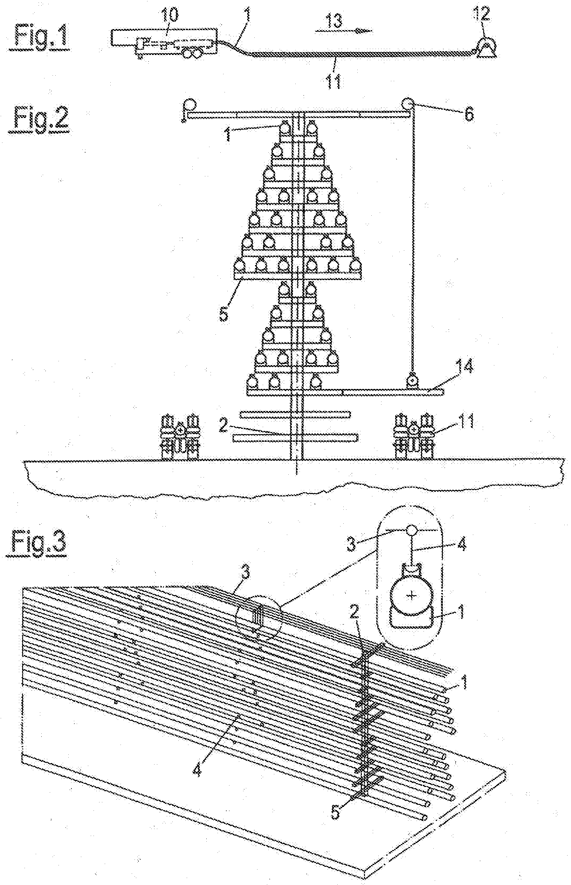

Im Interesse einer möglichst geringen Beanspruchung des Bioreaktor-Elementes 1 infolge Reibung wird dieses während der Herstellung zwischen Extruder 10 und dem Beginn des Bioreaktor-Elementes 1 auf einer Führungsvorrichtung 11 abgestützt, die sich, wie in Figur 1 dargestellt, über die gesamte Länge des fertigen Bioreaktor-Elementes 1 erstreckt.
Hierzu besitzt die Führungsvorrichtung 11 über die gesamte Länge verteilt eine Vielzahl von rotierenden Führungselementen in Form von Führungsrollen.
Diese Führungsrollen stützen das Bioreaktor-Element 1 von unten und führen dieses außerdem beidseitig bezüglich der Zugrichtung 13.

Somit kann das, den Extruder 10 verlassende Ende des Bioreaktor-Elementes 1 von dem Seil der Zugvorrichtung 12 in der Führungsvorrichtung 11 bis zur Endposition gezogen werden.

Um möglichst viel Biomasse produzieren zu können, umfasst die Anlage eine Vielzahl parallel verlaufender, sehr langgestreckter Bioreaktor-Elemente 1 mit einer zumindest teilweise transparenten Wandung aus Kunststoff, durch welche eine mit Mikroorganismen beimpfte Nährmittellösung zur Herstellung von Biomasse mittels Photosynthese geführt wird.
Damit der Aufwand für die Lagerung der Bioreaktor-Elemente 1 begrenzt bleibt, wird die entsprechende Stützvorrichtung für diese Bioreaktor-Elemente 1 von mehreren, entlang der Bioreaktor-Elemente 1 angeordneten und voneinander horizontal beabstandeten, feststehenden Stützmasten 2 gebildet.
Zwischen den Stützmasten 2 verlaufen über den jeweils zu tragenden Bioreaktor-Elementen 1 Tragelemente 3, die sich auf den entsprechenden Stützmasten 2 abstützen und mit den zu tragenden Bioreaktor-Elementen 1 über wenigstens ein Halteelement 4 an mindestens einem, zwischen zwei benachbarten Stützmasten 2 liegenden Abstützpunkt verbunden sind.
Auf diese Weise kann der Abstand zwischen den Stützmasten 2 sehr groß sein, ohne dass die Abstützung der Bioreaktor-Elemente 1 beeinträchtigt wird.

Selbst bei Bioreaktor-Elementen 1 mit einer Länge von 50 m oder mehr genügt es, wenn der Abstand zwischen zwei benachbarten Stützmasten 2 bei wenigstens 6 m und der Abstand zwischen zwei benachbarten Abstützpunkten zumindest 1 m beträgt.

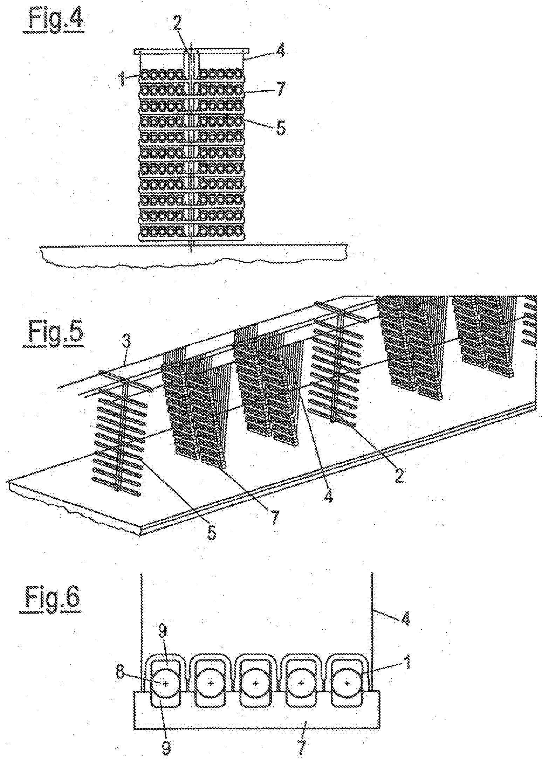

Die Tragelemente 3 erstrecken sich annähernd über die gesamte Länge der Bioreaktor-Elemente 1 und werden hier beispielhaft von einem flexiblen Stahlseil gebildet.
Die Haltelemente 4 können, wie in Figur 6 angedeutet, längenveränderbar oder aber ganz einfach als Haken geformt sein.

Eine derartige Stützkonstruktion ist nicht nur mit geringen Kosten sondern auch mit einem geringen Montageaufwand verbunden.

Neben der indirekten Abstützung über die Trag- 3 und Halteelemente 4 stützen sich die Bioreaktor-Elemente 1 auch direkt auf den Stützmasten 2, insbesondere auf, an den Stützmasten 2 befestigten Stützarmen 5 ab.

Für eine möglichst kompakte Anordnung sind die Bioreaktor-Elemente 1 in mehreren übereinander liegenden, horizontal verlaufenden Reihen angeordnet. Dabei stützt sich jede, auf einer Seite der Stützmasten 2 verlaufende Reihe von Bioreaktor-Elementen 1 auf jeweils einem Stützarm 5 der jeweiligen Stützmasten 2 ab.

Um eine Verbindung der Tragelemente 4 mit den Bioreaktor-Elementen 1 zu erleichtern, können die Bioreaktor-Elemente 1 gemäß den Figuren 2 und 3 im Bereich der Abstützpunkte Nuten, Ösen o.ä. aufweisen.
Außerdem zeigt Figur 2 noch Stützmasten 2 mit jeweils einer Hubvorrichtungen 6, welche geeignet sind, die Bioreaktor-Elemente 1 in die vorgesehene Höhe, insbesondere in den Bereich des entsprechenden Stützarmes 5 zu bringen.

Damit die Bioreaktor-Elemente 1 bei einer Montage nach Erreichen der erforderlichen Höhe einfach auf den entsprechenden Stützarm 5 geschoben werden können, sind die Stützarme 5 mittels Aufsatz 14 in den Hubbereich der Hubvorrichtungen 6 verlängerbar.

Je nach Belastbarkeit können jedem Bioreaktor-Element 1 ein eigenes oder mehreren Bioreaktor-Elementen 1 ein oder mehrere gemeinsame Tragelemente 3 zugeordnet sein.
Bei der Ausführung gemäß den Figuren 4 bis 6 sind mehrere benachbarte Bioreaktor-Elemente 1 im Bereich der Abstützpunkte über jeweils ein Koppelelement 7 miteinander verbunden. Die Koppelelemente 7 wiederum sind über jeweils zwei Halteelemente 4 an zwei Tragelementen 3 befestigt.

In ihrer einfachsten Ausführung haben die Bioreaktor-Elemente 1 nur einen Kanal 8 für die Nährmittellösung.
Zur Schaffung optimaler Temperaturbedingungen kann es aber notwendig sein, dass die Bioreaktor-Elemente 1 neben dem Haupt-Kanal 8 noch wie in Figur 2 und 3 einen oder gemäß den Figuren 4 bis 6 auch mehrere - hier zwei - Kühl-Kanäle 9 umfasst.

Mittels Extrusionsverfahren lassen sich auch Bioreaktor-Elemente 1 mit mehreren Kühl-Kanälen 9 einstückig herstellen.

## Patentansprüche

1. Stützvorrichtung mit einer Vielzahl parallel verlaufender, sehr langgestreckter Bioreaktor-Elemente (1) mit einer zumindest teilweise transparenten Wandung aus Kunststoff, durch welche eine mit Mikroorganismen beimpfte Nährmittellösung zur Herstellung von Biomasse mittels Photosynthese geführt wird, mit mehreren entlang der Bioreaktor-Elemente (1) angeordneten, horizontal voneinander beabstandeten, feststehenden Stützmasten (2), **dadurch gekennzeichnet, dass** über den jeweils zu tragenden Bioreaktor-Elementen (1) wenigstens ein Tragelement (3) zwischen zumindest zwei Stützmasten (2) verläuft, das Tragelement (3) sich auf den entsprechenden Stützmasten (2) abstützt, die zu tragenden Bioreaktor-Elemente (1) über wenigstens ein Halteelement (4) an mindestens einem, zwischen den beiden Stützmasten (2) liegenden Abstützpunkt mit zumindest einem Tragelement (3) verbunden sind und sich die Bioreaktor-Elemente (1) auf, an den Stützmasten (2) befestigten Stützarmen (5) abstützen.

2. Stützvorrichtung nach Anspruch 1, **dadurch gekennzeichnet, dass** die Stützmasten (2) Hubvorrichtungen (6) besitzen, welche geeignet sind, die Bioreaktor-Elemente (1) in die vorgesehene Höhe, insbesondere in den Bereich des entsprechenden Stützarmes (5) zu bringen.

3. Stützvorrichtung nach Anspruch 2, **dadurch gekennzeichnet, dass** die Stützarme (5) in den Hubbereich der Hubvorrichtungen (6) verlängerbar sind.

4. Stützvorrichtung nach einem der vorhergehenden Ansprüche, **dadurch gekennzeichnet, dass** die Ausdehnung der Halteelemente (4) veränderbar ist.

5. Stützvorrichtung nach einem der vorhergehenden Ansprüche, **dadurch gekennzeichnet, dass** über jedem Bioreaktor-Element (1) ein Tragelement (3) verläuft.

6. Stützvorrichtung nach einem der vorhergehenden Ansprüche, **dadurch gekennzeichnet, dass** an zumindest einem, vorzugsweise allen Tragelementen (3) jeweils mehrere Bioreaktor-Elemente (1) befestigt sind.

7. Stützvorrichtung nach einem der vorhergehenden Ansprüche, **dadurch gekennzeichnet, dass** die Bioreaktor-Elemente (1) im Bereich der Abstützpunkte Nuten, Ösen o.ä zur Verbindung mit dem entsprechenden Halteelement (4) aufweisen.

8. Stützvorrichtung nach einem der Ansprüche 1 bis 6, **dadurch gekennzeichnet, dass** mehrere benachbarte Bioreaktor-Elemente (1) im Bereich wenigstens eines Abstützpunktes über jeweils ein Koppelelement (7) miteinander verbunden sind und das Koppelelement (7) über ein oder mehrere Halteelemente (4) an einem oder mehreren Tragelementen (3) befestigt ist.

9. Stützvorrichtung nach einem der vorhergehenden Ansprüche, **dadurch gekennzeichnet, dass** sich die Tragelemente (3) zumindest annähernd über die gesamte Länge der Bioreaktor-Elemente (1) erstrecken.

10. Stützvorrichtung nach einem der vorhergehenden Ansprüche, **dadurch gekennzeichnet, dass** die Bioreaktor-Elemente (1) in mehreren übereinander liegenden, vorzugsweise horizontal verlaufenden Reihen angeordnet sind.

11. Stützvorrichtung nach Anspruch 10, **dadurch gekennzeichnet, dass** sich zumindest eine, vorzugsweise jede auf einer Seite des Stützmasten (2) verlaufende Reihe von Bioreaktor-Elementen (1) auf jeweils einem Stützarm (5) des Stützmasten (2) abstützt.

12. Stützvorrichtung nach einem der vorhergehenden Ansprüche, **dadurch gekennzeichnet, dass** die Bioreaktor-Elemente (1) eine Länge von mehr als 25, vorzugsweise mehr als 50 und insbesondere mehr als 100 m haben und/oder der Abstand zwischen zwei benachbarten Stützmasten (2) mindestens 3, vorzugsweise wenigstens 6 m und/oder der Abstand zwischen zwei benachbarten Abstützpunkten zumindest 1, vorzugsweise wenigstens 2 m beträgt.

13. Stützvorrichtung nach einem der vorhergehenden Ansprüche, **dadurch gekennzeichnet, dass** die Bioreaktor-Elemente (1) jeweils einstückig ausgebildet sind.

14. Stützvorrichtung nach einem der vorhergehenden Ansprüche, **dadurch gekennzeichnet, dass** das Tragelement (3) als flexibles Stützseil, Stützband o.ä. ausgebildet ist.

## Claims

1. Supporting device for a multiplicity of highly elongate bioreactor elements (1) running in parallel and having an at least partially transparent wall of plastic, through which a nutrient solution inoculated with microorganisms is conducted for producing biomass by means of photosynthesis, having a plurality of stationary supporting pylons (2) arranged along the bioreactor elements (1) and horizontally spaced apart from one another, **characterized in that** at least one carrying element (3) runs between at least two supporting pylons (2), over the bioreactor elements (1) respectively to be carried, which carrying element (3) is supported on the corresponding supporting pylons (2), the bioreactor elements (1) to be carried are connected to at least one carrying element (3) by way of at least one holding element (4) at at least one supporting point lying between the two supporting pylons (2) and on supporting arms (5) fastened to the supporting pylons (2).

2. Supporting device according to Claim 1, **characterized in that** the supporting pylons (2) have lifting devices (6) suitable for bringing the bioreactor elements (1) to the intended height, in particular into the region of the corresponding supporting arm (5).

3. Supporting device according to Claim 2, **characterized in that** the supporting arms (5) are extendable into the lifting region of the lifting devices (6).

4. Supporting device according to one of the preceding claims, **characterized in that** the extent of the holding elements (4) is variable.

5. Supporting device according to one of the preceding claims, **characterized in that** a carrying element (3) runs over each bioreactor element (1).

6. Supporting device according to one of the preceding claims, **characterized in that** a plurality of bioreactor elements (1) are respectively fastened to at least one carrying element (3), preferably all of the carrying elements (3).

7. Supporting device according to one of the preceding claims, **characterized in that** the bioreactor elements (1) have grooves, eyelets or the like in the region of the supporting points, for connection to the corresponding holding element (4).

8. Supporting device according to one of Claims 1 to 6, **characterized in that** a plurality of neighboring bioreactor elements (1) are connected to one another in the region of at least one supporting point by way of a coupling element (7) in each case, and the coupling element (7) is fastened to one or more carrying elements (3) by way of one or more holding elements (4).

9. Supporting device according to one of the preceding claims, **characterized in that** the carrying elements (3) extend at least almost over the entire length of the bioreactor elements (1).

10. Supporting device according to one of the preceding claims, **characterized in that** the bioreactor elements (1) are arranged in a plurality of rows that lie one above the other and preferably run horizontally.

11. Supporting device according to Claim 10, **characterized in that** at least one, preferably each, row of bioreactor elements (1) running on one side of the supporting pylons (2) is supported on a supporting arm (5) in each case of the supporting pylons (2).

12. Supporting device according to one of the preceding claims, **characterized in that** the bioreactor elements (1) have a length of over 25, preferably over 50 and in particular over 100 m and/or the distance between two neighboring supporting pylons (2) is at least 3, preferably at least 6 m and/or the distance between two neighboring supporting points is at least 1, preferably at least 2 m.

13. Supporting device according to one of the preceding claims, **characterized in that** the bioreactor elements (1) are in each case formed in one piece.

14. Supporting device according to one of the preceding claims, **characterized in that** the carrying element (3) is formed as a flexible supporting cable, supporting band or the like.

## Revendications

1. Dispositif de support avec une multiplicité d'éléments de bioréacteur (1) très allongés s'étendant parallèlement, avec une paroi au moins partiellement transparente en matière plastique, à travers laquelle on conduit une solution nutritive inoculée avec des microorganismes pour la production de biomasse par photosynthèse, avec plusieurs mâts de support fixes (2), espacés horizontalement l'un de l'autre et disposés le long des éléments de bioréacteur (1), **caractérisé en ce qu'**au moins un élément porteur (3) s'étend au-dessus des éléments de bioréacteur à porter (1) entre au moins deux mâts de support (2), l'élément porteur (3) prend appui sur les mâts de support correspondants (2), les éléments de bioréacteur à porter (1) sont reliés à au moins un élément porteur (3) par au moins un élément de retenue (4) en au moins un point d'appui situé entre les deux mâts de support (2) et les éléments de bioréacteur (1) s'appuient sur des bras de support (5) fixés aux mâts de support (2).

2. Dispositif de support selon la revendication 1, **caractérisé en ce que** les mâts de support (2) comportent des dispositifs de levage (6), qui conviennent pour amener les éléments de bioréacteur (1) à la hauteur prévue, en particulier dans la région du bras de support correspondant (5).

3. Dispositif de support selon la revendication 2, **caractérisé en ce que** les bras de support (5) peuvent être prolongés dans la région de levage des dispositifs de levage (6).

4. Dispositif de support selon l'une quelconque des revendications précédentes, **caractérisé en ce que** l'extension des éléments de retenue (4) est variable.

5. Dispositif de support selon l'une quelconque des revendications précédentes, **caractérisé en ce qu'**un élément porteur (3) court au-dessus de chaque élément de bioréacteur (1).

6. Dispositif de support selon l'une quelconque des revendications précédentes, **caractérisé en ce que** plusieurs éléments de bioréacteur (1) sont chaque fois fixés à au moins un, de préférence à tous les élément(s) porteur(s) (3).

7. Dispositif de support selon l'une quelconque des revendications précédentes, **caractérisé en ce que** les éléments de bioréacteur (1) présentent dans la région des points d'appui des rainures, des oreilles ou analogues pour la liaison à l'élément de retenue correspondant (4).

8. Dispositif de support selon l'une quelconque des revendications 1 à 6, **caractérisé en ce que** plusieurs éléments de bioréacteur voisins (1) sont assemblés l'un à l'autre dans la région d'au moins un point d'appui chaque fois par un élément de couplage (7) et l'élément de couplage (7) est fixé à un ou plusieurs élément (s) porteur(s) (3) au moyen d'un ou de plusieurs élément(s) de retenue (4).

9. Dispositif de support selon l'une quelconque des revendications précédentes, **caractérisé en ce que** les éléments porteurs (3) s'étendent au moins approximativement sur toute la longueur des éléments de bioréacteur (1).

10. Dispositif de support selon l'une quelconque des revendications précédentes, **caractérisé en ce que** les éléments de bioréacteur (1) sont disposés en plusieurs rangées disposées l'une au-dessus de l'autre, qui courent de préférence horizontalement.

11. Dispositif de support selon la revendication 10, **caractérisé en ce qu'**au moins une, de préférence chaque rangée d'éléments de bioréacteur (1) s'étendant sur un côté du mât de support (2) prend appui respectivement sur un bras de support (5) du mât de support (2).

12. Dispositif de support selon l'une quelconque des revendications précédentes, **caractérisé en ce que** les éléments de bioréacteur (1) ont une longueur de plus de 25, de préférence de plus de 50, et en particulier de plus de 100 m et/ou la distance entre deux mâts de support voisins (2) vaut au moins 3, de préférence au moins 6 m et/ou la distance entre deux points d'appui voisins vaut au moins 1, de préférence au moins 2 m.

13. Dispositif de support selon l'une quelconque des revendications précédentes, **caractérisé en ce que** les éléments de bioréacteur (1) sont chaque fois réalisés en une seule pièce.

14. Dispositif de support selon l'une quelconque des revendications précédentes, **caractérisé en ce que** l'élément porteur (3) est réalisé sous forme de câble de support, bande de support ou analogue.
